(19) Europäisches Patentamt
European Patent Office
Office européen des brevets

(11) **EP 3 041 495 B1**

(12) **EUROPÄISCHE PATENTSCHRIFT**

(45) Veröffentlichungstag und Bekanntmachung des Hinweises auf die Patenterteilung:
**06.03.2019 Patentblatt 2019/10**

(51) Int Cl.:
**A61K 38/42** *(2006.01)*    **A61P 11/00** *(2006.01)*

(21) Anmeldenummer: **14758344.7**

(22) Anmeldetag: **27.08.2014**

(86) Internationale Anmeldenummer:
**PCT/EP2014/068185**

(87) Internationale Veröffentlichungsnummer:
**WO 2015/032672 (12.03.2015 Gazette 2015/10)**

(54) **ZUBEREITUNGEN ZUR VERBESSERTEN GEWEBE-OXYGENIERUNG DURCH PERITONEALE BEATMUNG**

PREPARATIONS FOR IMPROVED TISSUE OXYGENATION BY MEANS OF PERITONEAL VENTILATION

PRÉPARATIONS POUR UNE MEILLEURE OXYGÉNATION DES TISSUS PAR RESPIRATION PÉRITONÉALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.09.2013 DE 102013014651**

(43) Veröffentlichungstag der Anmeldung:
**13.07.2016 Patentblatt 2016/28**

(73) Patentinhaber: **SanguiBioTech GmbH 58455 Witten (DE)**

(72) Erfinder:
- **PÖTZSCHKE, Harald 65191 Wiesbaden (DE)**
- **SCHMELZ, Hubertus 22587 Hamburg (DE)**

(74) Vertreter: **Müller, Claudia European Patent Attorney Uhlandstrasse 58 60314 Frankfurt / Main (DE)**

(56) Entgegenhaltungen:
**EP-A2- 0 209 128    WO-A1-03/094953 WO-A2-03/082392**

- **NORIYUKI MATSUTANI ET AL: "THE PERITONEUM AS A NOVEL OXYGENATION ORGAN", SHOCK, 2008, Seite 1, XP055145077, ISSN: 1073-2322, DOI: 10.1097/shk.0b013e318162be0a**
- **NORIYUKI MATSUTANI ET AL: "Efficacy of peritoneal oxygenation using a novel artificial oxygen carrier (TRM-645) in a rat respiratory insufficiency model", SURGERY TODAY ; OFFICIAL JOURNAL OF THE JAPAN SURGICAL SOCIETY, SPRINGER-VERLAG, TO, Bd. 40, Nr. 5, 28. April 2010 (2010-04-28) , Seiten 451-455, XP019804781, ISSN: 1436-2813**

**Beschreibung**

Gegenstand der Erfindung

Die vorliegende Erfindung betrifft den Bereich der (trans-)peritonealen Beatmung eines Säugetieres, insbesondere eines Menschen, zur zusätzlichen Sauerstoffversorgung des Organismus oder einzelner Organe im insbesondere lebensbedrohlichen Bedarfsfall. Dieser liegt vor allem bei einer Unterversorgung des Blutes und infolgedessen des Körpers mit Sauerstoff durch ein Lungenversagen vor. Darüber hinaus gibt es auch Sauerstoffmangel einzelner Bauchorgane, wie z. B. bei einem Kreislaufschock (hypovolämisch, septisch, etc). Derartige Zustände können durch eine transperitoneale Verabreichung von Sauerstoff aus einem in die Bauchhöhle eingebrachten Gas bekämpft werden (so genannte peritoneale oder transperitoneale Beatmung), wobei erfindungsgemäß zusätzlich eine flüssige, vor allem wässrige, einen Sauerstoffbinder wie Hämoglobin enthaltende Zubereitung eingesetzt wird. Eine derartige, einen Sauerstoffbinder enthaltende flüssige Zubereitung oder Lösung vermag die Effektivität der Diffusion des durch (einmalige oder mehrfache) Befüllung oder kontinuierliche Spülung der Bauchhöhle verabreichten Sauerstoffs in unerwarteter Weise effektiv und auf schnelle und einfache Weise zu steigern. Dadurch kann die peritoneale Ventilation verbessert und eine Hypoxie im Körper wirksamer behandelt werden.

Die Erfindung betrifft insofern auch die An- bzw. Verwendung eines Sauerstoffbinders, insbesondere ausgewählt aus Hämoglobin, Myoglobin, Hämocyanin, Erythrocruorin, oder Derivaten hiervon, vor allem von nativem Schweinehämoglobin, insbesondere mit einem Halbsättigungs-Sauerstoffpartialdruck zwischen 1 und 50 Torr, zur Herstellung einer vor allem wässrigen Zubereitung zur Verstärkung der Diffusion von in Schleimschichten der Bauchfellschleimhaut eingebrachtem Sauerstoff(gas), insbesondere bei der transperitonealen Beatmung.

Stand der Technik

Bei einem Lungenversagen - bspw. ALI (Acute Lung Injury) und ARDS (Acute Respiratory Distress Syndrom) - ist dort u. a. die Sauerstoff-Aufnahme und Versorgung des Blutes mit frischem Sauerstoff (Oxygenierung des Blutes) beeinträchtigt. Dies führt zu einem Sauerstoffmangel im Blut (Hypoxämie) und nachfolgend im Körper (systemische Hypoxie, mangelnde Oxygenierung der Gewebe). Um Schäden und schließlich den Tod des Betroffenen zu verhindern, muss ihm therapeutisch Sauerstoff zugeführt werden. Bei leichten Fällen kann es reichen, den Sauerstoffgehalt in der Atemluft zu steigern, bei schwereren Fällen kann zusätzlich mit (endexspiratorischem) Überdruck beatmet werden.

Als letzte Maßnahme kann das Blut außerhalb des Körpers in einer technischen Vorrichtung (Oxygenator) mit Sauerstoff beladen (oxygeniert) werden. Technische Oxygenatoren (z. B. Extrakorporale Membran-Oxygenatoren: ECMO) werden auch zu Operationen z. B. am Herzen oder der Lunge eingesetzt (z. B. im Rahmen einer so genannten Herz-Lungen-Maschine). Ihr Einsatz ist sehr aufwändig und anspruchsvoll, zugleich auch sehr risikoreich. Außerdem gibt es Krankheitsbilder, die mit einer verminderten Oxygenierung der Organe im Bauchraum, also einer lokalen Hypoxie, einhergehen, wie bspw. hypovolämischer oder septischer Kreislaufschock, Darminfarkte, etc., oder solche, die eine vorübergehende therapeutische Steigerung der Oxygenierung von Organen im Bauchraum erfordern (z. B. die Implantation von Insulin-produzierenden Langerhansschen Zellen in die Leber, etc.), bei denen eine lokale Verbesserung der Oxygenierung medizinisch wünschenswert ist.

Es wurden etliche Versuche unternommen, neue praktikable Verfahren zur Behebung einer systemischen oder lokalen Hypoxie zu entwickeln, um insbesondere Risiken und aufwendige technische Vorrichtungen, welche im Bedarfsfall ggf. nicht vorhanden sind, zu vermeiden. So wurde als eine alternative Maßnahme einer nicht-pulmonalen Lungen-Unterstützung (pulmonary support) eine therapeutische Sauerstoff-Versorgung über die Schleimhaut der Bauchhöhle (das Bauchfell (Peritoneum), eine Serosa) erwogen.

Eine prinzipielle Möglichkeit einer (trans-)peritonealen Oxygenierung besteht darin, den Bauchraum mit einer Sauerstoff-reichen Flüssigkeit (diese enthält bereits gelösten Sauerstoff) einmalig oder wiederholt zu füllen oder fortlaufend zu spülen. Entsprechende Zubereitungen sind oder enthalten z. B. mit Sauerstoff übersättigte wässrige Salzlösungen, oder insbesondere auch oxygenierte (Per-)Fluorocarbone. Die Patente US 4,657,532 und US 4,963,130 sowie EP 0 209 128 B1 beschreiben ein derartiges Verfahren der Befüllung der Bauchhöhle mit flüssigen Sauerstoff-beladenen Fluorocarbonverbindungen oder mit Emulsionszubereitungen, die diese enthalten.

Eine andere Methode ist die Anwendung von Sauerstoff-haltigem Gas in Form einer peritonealen Beatmung. Diese ist z. B. von Zhang J.-Y., et al. in "Effect of Oxygenation of Transperitoneal Ventilation on the Death Time After Asphyxiation in Rabbits", Minerva Anesthesiologica 2010; 76: 913 - 918 anhand eines Experimentes mit Kaninchen beschrieben, wobei feststellbare Mengen an Sauerstoff in den Körper der Tiere gelangten (und die Überlebenszeiten deutlich verlängert wurden).

Im Unterschied dazu berichten Giffin D.M., et al. in "Oxygen Uptake During Peritoneal Ventilation in a Porcine Model of Hypoxemia", Critical Care Medicine 1998; 26: 1564 - 1568, am Modell narkotisierter Schweine, dass bei diesen größeren Organismen keine signifikante Zunahme der Sauerstoff-Aufnahme durch peritoneale Beatmung erfolgt.

Eine klinische Anwendungsreife hat keine der angeführten experimentellen Methoden erlangt. Auch kann kein allgemeines Verfahren aufgrund der unterschiedlichen und teilweise widersprüchlichen Ergebnisse hieraus abgeleitet werden.

Aufgabe der Erfindung

Der Erfindung liegt die Aufgabe zugrunde, hypoxische Zustände von Organismen (insbesondere Säugetierorganismen) oder einzelner Organe dieser auf einfache und effektive Weise durch eine (trans-)peritoneale Beatmung zu verbessern. Dabei soll der klinische Nutzen die Risiken deutlich überwiegen und aufwendige Technik vermieden werden.

Lösung der Aufgabe

Diese Aufgabe wird erfindungsgemäß dadurch gelöst, dass man eine (trans-)peritoneale Beatmung mit sauerstoffhaltigem Gas anwendet und dabei eine flüssige, einen Sauerstoffbinder, insbesondere ein Sauerstoff bindendes Protein, vor allem Hämoglobin, enthaltende Zubereitung zur Diffusionsverstärkung einsetzt. Die den Sauerstoffbinder, insbesondere Hämoglobin enthaltende Zubereitung (Lösung) wird im insbesondere hypoxischen Bedarfsfall, - entweder vor oder bei gleichzeitiger Begasung - auf das Peritoneum aufgebracht und die peritoneale Ventilation mit einem Sauerstoff-haltigen Gas wie bekannt durchgeführt. Überraschenderweise hat sich gezeigt, dass die transperitoneale Oxygenierung von Säugetieren, insbesondere auch des Menschen, durch eine verbesserte Sauerstoffdiffusion durch das Bauchwasser zum Peritoneum mit der flüssigen Zubereitung verbessert werden kann. Damit kann vor allem die Oxygenierung der Darm- und Magenwand, sowie der Oberflächen der Organe mit Peritoneum wie vor allem Bauchwand, großes Netz, Gekröse, Harnblase, Retroperitonealraum-Organe, Leber, Bauchspeicheldrüse, etc. einerseits lokal gesteigert werden. Dies kann andererseits insgesamt über das dort fließende Blut auch zur gesteigerten Sauerstoffversorgung des gesamten Organismus systemisch genutzt werden. Somit wird erfindungsgemäß auf einfache und effektive Art die (trans-)peritoneale Oxygenierung durch eine (trans)-peritoneale Beatmung sowohl lokal als auch systemisch gesteigert.

Die erfindungsgemäß eingesetzte Zubereitung führt zu einer verbesserten peritonealen Beatmung. Diese kann somit zu einer klinisch sinnvoll einsetzbaren Methode genutzt werden, und Patienten retten oder deren Heilung fördern.

Dies ist deshalb überraschend, weil zwar bekannt war, dass Sauerstoffbinder wie Hämoglobin Sauerstoff reversibel zu binden (so genannte Protein-Oxygenierung) und dadurch den Transport von Sauerstoff in wässrigen Lösungen zu steigern vermögen (Mechanismus der erleichterten Diffusion). Allerdings hängt diese Diffusion auch von der Beschaffenheit des zu diffundieren Mediums ab: So stellen Wasser bzw. wässrige Medien selbst bereits Diffusionssperren für Sauerstoff dar, da Sauerstoff in Wasser nur sehr schwer löslich ist. Bereits eine dünne Wasserschicht ist eine effektive Sperre für den Transport von Sauerstoff durch Diffusion. Die Bauchhöhlenorgane sind jedoch nicht nur mit Wasser, sondern mit einer dünnen wässrigen Schleimlösung überzogen. Das gesamte Volumen dieser Peritonealflüssigkeit beträgt beim gesunden Menschen etwa 50 - 100 mL. Diese einerseits für die Verschieblichkeit der inneren Organe erforderliche Schicht ist andererseits eine erhebliche Diffusionssperre für Sauerstoff und behindert den Durchtritt von Sauerstoff bei der peritonealen Beatmung. Um so überaschender war es, dass durch die Anwendung einer flüssigen, ebenfalls diffusionsabhängigen Sauerstoff-haltigen Lösung die Diffusion des bei der peritonealen Beatmung verabreichten gasförmigen Sauerstoffs aus dem Bauchraum in die Gewebe (z. B. Magen, Darm, Leber) verbessert wird.

Nähere Erläuterung der Erfindung

Erfindungsgemäß werden insbesondere hoch-konzentrierte flüssige Zubereitungen in Form wässriger Lösungen eingesetzt, enthaltend mindestens einen Sauerstoffbinder, insbesondere ein Sauerstoff-bindendes Protein, wie vor allem Hämoglobin, Myoglobin, Hämocyanin, oder Erythrocruorin, etc., oder ein durch chemische Modifikation erzeugtes Derivat hiervon, oder Mischungen mehrerer derartiger Sauerstoffbinder, als Diffusionsverstärker von Sauerstoff-haltigem Gas bei der peritonealen Beatmung. Erfindungsgemäß wird die Zubereitung so eingesetzt, dass die freie Oberfläche der Bauchhöhlenorgane (das Peritoneum) möglichst umfänglich mit der genannten Lösung dünn benetzt wird. Dazu können bekannte Vorrichtungen und Vorgehensweisen verwendet werden, wie sie z. B. zur Spülung einer Bauchhöhle, oder zur nachfolgenden peritonealen Beatmung verwendet werden. Diese bestehen im Prinzip aus einem flexiblen oder starren Katheder (Tubus oder Kanüle) und einer scharfen Füllung (Draht oder Stift) zum Durchstich durch die Bauchwand (Trokar oder scharfer Mandrin).

Auch ein Wechsel des Katheders, z. B. Einstich mit einem starren und nachfolgender Ersatz durch einen biegsamen Katheter, z. B. nach der Seldinger-Technik, ist möglich. Die flüssige, den/die Sauerstoffbinder enthaltende Zubereitung wird auf diese oder analoge Weise in die Bauchhöhle des Säugetiere injiziert und somit verteilt bzw. dadurch das Peritoneum benetzt. Vorzugsweise danach erfolgt die Beatmung mit dem Sauerstoff-haltigen Gas. Das Sauerstoff-haltige Gas ist insbesondere reiner Sauerstoff, oder mit Sauerstoff angereicherte Luft oder ein Sauerstoff-Inertgas-Gemisch wie vor allem ein Stickstoff-Sauerstoff-Gemisch mit mindestens 25 Vol.-% Sauerstoff. Es kann als entspanntes Druckgas oder über geeignete Pumpvorrichtungen über die gleichen Katheter wie die erfindungsgemäße flüssige Zubereitung eingefüllt werden. Die Geschwindigkeit des Gaseintrages erfolgt wie bekannt.

Bei einem Wirkungsverlust der Lösung des Diffusionsverstärkers kann dieser periodisch neu verabreicht werden, z. B. alle 2 oder 4 oder 8 Stunden, etc.

Zur Entfernung überflüssiger oder störender Mengen wässriger Lösung im Bauchraum kann dieser in bekannter Weise mit einer Spüllösung gespült und entleert werden, bevor die Lösung des Diffusionsverstärkers erneut verabreicht wird.

Diese Wirkung einer einen Sauerstoffbinder wie vor allem Hämoglobin enthaltenden Lösung als Diffusionsverstärker war nicht zu erwarten, da wässrige Flüssigkeiten wie erwähnt prinzipiell Diffusionssperren für Sauerstoff sind. Überraschender Weise kann diese jedoch erfindungsgemäß überwunden werden, indem die beschriebene Zubereitung in einer insbesondere geringen Menge aufgetragen wird, die nur eine Benetzung der Oberfläche des Peritoneums bewirkt. Dazu ist die erfindungsgemäße Zubereitung vor allem hochkonzentriert an Sauerstoffbinder. Nach der Mischung mit dem vorhandenen Bauchwasser (Peritonealflüssigkeit) übersteigt in der resultierenden, weiterhin noch dünnen Schicht die zusätzliche erleichterte Diffusion durch den Sauerstoffträger (Sauerstoffbinder) die zusätzliche Verminderung der einfachen (freien) Diffusion von Sauerstoff in der zusätzlichen Schichtdicke der wässrigen Lösung.

Die Erfindung ist auch deshalb überraschend, weil angenommen werden muss, dass höhere Mengen an flüssiger, den diffusionsverstärkenden Sauerstoffbinder enthaltenden Zubereitung vermutlich den Übertritt von Sauerstoff aus dem Beatmungs-Gas in das Gewebe wieder vermindern können, da dann die Diffusionsstrecken für Sauerstoffbinder wie z.B. Hämoglobin in der dann dichteren wässrigen Schicht zu lang werden.


Ausführliche Beschreibung der Erfindung


1. Komponenten der Sauerstoffbinder-haltigen Zubereitung und deren Herstellung


Die erfindungsgemäß eingesetzte flüssige Zubereitung basiert vor allem auf Wasser, z. B. gereinigtem Wasser für Injektionszwecke, und enthält mindestens einem Sauerstoffbinder, (Atmungsprotein). Auch Kombinationen der nachfolgend genannten Sauerstoffbinder sind möglich. Dessen (oder deren) Konzentration beträgt vorteilhafter Weise gleich oder mehr als 100 g/L, vor allem 150 bis 400 g/L, insbesondere 200 bis 350 g/L, vor allem 250 bis 300 g/L. Bevorzugte Konzentrationen liegen zwischen 150 und 400 g/L, vor allem zwischen 200 und 350 g/L und ganz besonders bevorzugt zwischen 250 und 300 g/L.

Als Sauerstoffbinder eignen sich vor allem solche höherer Organismen wie Hämoglobin, Myoglobin, insbesondere Hämoglobin und Myoglobin von Säugern wie vor allem vom Schwein, Menschen, Rind, Schaf, Pferd, oder Ziege, etc. Als Sauerstoffbinder sind auch Sauerstoff-Transportproteine niederer Tiere, wie Hämocyanine, oder Erythrocruorine, etc. geeignet. Der oder die Sauerstoffbinder sind bevorzugt nativ, also chemisch nicht modifiziert. Aber auch chemisch modifizierte Derivate der genannten Sauerstoffbinder können verwendet werden. Die chemische Modifikation kann z. B eine Vernetzung, Polymerisation, kovalente Addition eines weiteren Moleküls (z. B. eines Polyalkylenoxids oder eines Effektors) oder eine Kombination einer oder mehrerer dieser Modifikationen sein, wobei prinzipiell auch eine weitere, hier nicht genannte chemische Modifikation inkludiert sein soll. Die Vernetzung monomeren, nativen oder mit Effektoren verknüpften Hämoglobins oder Myoglobins mit etlichen Vernetzern sind bekannt und beispielsweise mit Divinylsulfon, Epichlorhydrin, Butadiendiepoxid, Hexamethylendiisocyanat, Dialdehyden wie Glyoxal und Glutardialdehyd, Diimidoestern wie Dimethylsuberimidat, Dimethylmalonimidat und Dimethyladipimidat, etc. beschrieben. Zur Pegylierung sind z. B. Polyethylenoxid, Polypropylenoxid, oder Kopolymere aus Ethylenoxid und Propylenoxid, insbesondere Ester oder Ether hiervon mit einem kurzkettigen aliphatischen organischen Rest geeignet. Ein kovalent angeknüpftes Polyalkylenoxid oder sonstiges Makromolekül kann eine molare Masse zwischen 200 und 10.000 g/mol aufweisen.

Derartige Sauerstoffbinder und Derivate hiervon sind bekannt und beispielweise in der DE 100 31 740 A1, DE-100 31 744 A1 und der DE 100 31 742 A1 beschrieben und können eine Molmasse von 70.000 bis 15.000.000 g/mol aufweisen.

Derartige Verfahren können auch auf andere Sauerstoffbinder angewendet werden.

Der Sauerstoffbinder, insbesondere Hämoglobin oder Myoglobin, kann gegebenenfalls zur verbesserten Lagerfähigkeit durch Karbonylierung desoxygeniert sein. Insbesondere sind die Sauerstoffbindungsstellen mit Kohlenmonoxid gesättigt, so dass das Mittel Kohlenmonoxid enthält. Zur Anwendung kann er wahlweise oxygeniert werden, oder es wird in der Bauchhöhle durch das Beatmungsgas (innert weniger Minuten) oxygeniert. Derartige Karbonylierungsverfahren sind bekannt.

Ganz besonders bevorzugt weisen die erfindungsgemäß eingesetzten Sauerstoffbinder der beschriebenen Art einen Sauerstoff-Halbsättigungs-Partialdruck p50 zwischen 1 und 50 Torr auf. Der Sauerstoff-Halbsättigungs-Partialdruck p50 ist dabei der Sauerstoff-Partialdruck, bei dem der Sauerstoff-Träger hälftig Sauerstoff gebunden hat.

Besonders bevorzugt wird natives Hämoglobin oder natives Myoglobin vom Menschen, vom Schwein oder vom Rind eingesetzt. Derartige Sauerstoffbinder können vor allem auch karbonyliert sein, wie oben beschrieben.

Die bevorzugte Menge an einzusetzendem Sauerstoffbinder zur Erzielung einer größtmöglichen diffusionsverstärkenden Wirkung kann ermittelt werden unter Bezugnahme auf die von J. B. Wittenberg ("The Molecular Mechanism of Haemoglobin-facilitated Oxygen Diffusion", Journal of Biological Chemistry 1966; 241: 104 - 114) experimentell für reale, rein wässrige Verhältnisse (wässrige Lösung, Hämoglobin) ermittelte Menge. Wittenberg beschreibt für eine Hämoglobin-Konzentration von etwa 100 g/L ein Maximum der erleichterten Diffusion von Di-sauerstoff durch dünne Schichten wässriger Lösungen.

Unter Bezugnahme auf diese empirisch ermittelten Verhältnisse werden daher erfindungsgemäß flüssige, einen oder mehrere Sauerstoffbinder enthaltende Zubereitungen zur Verbesserung einer transperitonealen Beatmung bei einem Säugetier, insbesondere Menschen, in einer Menge eingesetzt, welche einen Gehalt hiervon in der Peritonealflüssigkeit um etwa 100 g/L ergeben.

Die benötigte Menge (Volumen) einer einen (oder mehrere) Sauerstoffbinder, insbesondere Hämoglobin, enthaltenden Zubereitungslösung ($V_{HL}$) zur Erzielung der größtmöglichen Wirkung kann demnach ermittelt werden gemäß der Gleichung:

$$V_{HL} = (100 \text{ g/L} \bullet V_{BW}) / (c_{HL} - 100 \text{ g/L})$$

$V_{BW}$ ist das Volumen des Bauchwassers (etwa 50 - 100 mL) und $C_{HL}$ die Hämoglobin-Konzentration der zu verabreichenden Hämoglobin-Lösung.

Hieraus ergibt sich beispielsweise, dass man für 50 mL Bauchwasser und eine Hämoglobinlösung der Konzentration 400 g/L 16,7 mL benötigt. Bei 100 mL Bauchwasser und einer Hämoglobinlösung der Konzentration 200 g/L sind es 100 mL. Diese Berechnung kann mutatis mutandis auch für andere Sauerstoffbinder/Derivate herangezogen werden.

Die wässrige Zubereitung kann weiterhin einen oder mehrere Zusätze enthalten, ausgewählt aus Salzen, insbesondere der Gruppe, umfassend, vor allem bestehend aus, Natrium- , Kalium-, Kalzium- und Magnesium-Ionen als Kationen, sowie Chlorid- , (Hydrogen-)-Carbonat-, (Hydrogen-)-Phosphat-, Citrat- und Lactat-Ionen als Anionen, sowie geeigneten Konservierungsmitteln, jeweils in einer für die betreffende Anwendung geeigneten Konzentration. Dies sind z. B. Gehalte, die den in der Extrazellular-Flüssigkeit des Menschen (oder des peritoneal beatmeten Tieres) gegebenen Gehalten entsprechen oder nahe kommen (so genannte physiologische Lösungen), oder die Gehalte sonstiger, empirisch als verträglich bekannter Infusionslösungen oder Lösungen zur Peritoneal-Lavage oder Peritoneal-Dialyse. Der pH-Wert der Diffusionsverstärkerlösung wird insbesondere durch Titration des Hämoglobins, Myoglobins oder sonstigen Sauerstoffbinders (Atmungsproteins) auf einen geeigneten pH-Wert, z.B. zwischen 7,0 und 7,7 eingestellt. Bevorzugt ist der der pH-Wert der erfindungsgemäßen Diffusionsverstärkerlösung auf einen physiologischen Wert um 7,4 eingestellt.

Die Zubereitungen werden durch aseptische Prozesse hergestellt, indem der oder die Sauerstoffbinder aus den biologischen Quellen extrahiert und aufgereinigt, ggf. chemisch modifiziert, und durch Lösungmitteltausch in eine der oben beschriebenen wässrigen Lösungen umgewaschen wird. Diese Zubereitungen können sodann in geeignete Ampullen oder in für Injektionszwecke geeignete Vorrichtungen zum sofortigen Gebrauch abgefüllt werden.

Die Erfindung betrifft daher auch ein Verfahren zur Herstellung einer Zubereitung zur Diffusionsverstärkung für Sauerstoffgas im Bauchwasser zur Verbesserung der Oxygenierung durch peritoneale Beatmung eines Säugetiers im hypoxischen Bedarfsfall, insbesondere zur systemischen Verbesserung der Oxygenierung eines Säugetiers durch peritoneale Beatmung, das dadurch gekennzeichnet ist, dass man einen oder mehrere Sauerstoffbinder, ausgewählt aus Hämoglobin, Myoglobin, Hämocyanin, Erythrocruorin, oder Derivaten oder Mischungen hiervon aus biologischen Quellen, insbesondere Tierblut der o.g. Tiere in an sich bekannter Weise extrahiert und aufreinigt und sodann ggf. nach chemischer Modifikation, durch Lösungsmitteltausch in eine wässrige Lösung, wahlweise enthaltend Zusatzstoffe, ausgewählt aus Salzen, Konservierungsmitteln oder Mischungen hiervon, umwäscht und vorzugsweise auf einen pH-Wert von 7 bis 7,7 einstellt, und insbesondere die Zubereitung in geeignete Ampullen oder in für Injektionszwecke geeignete Vorrichtungen zum sofortigen Gebrauch abfüllt.

So können die erfindungsgemäß eingesetzten flüssigen Zubereitungen für den Einsatz zur Verbesserung der peritonealen Beatmung hergestellt werden, wobei der oder die Sauerstoffbinder in der angegebenen Konzentration, nämlich von 150 bis 400 g/L, bevorzugt von 200 bis 350 g/L und ganz besonders bevorzugt von 250 bis 300 g/L, vor allem zusammen mit einem physiologischen Natriumchloridgehalt in Wasser vorliegt/en.

Als Sauerstoffbinder eignen sich vor allem solche höherer Organismen wie Hämoglobin.

Bevorzugte Ausgestaltungen der Erfindung umfassen folgende Zubereitungen und deren Anwendungen:

- Zubereitungen, worin als Sauerstoffbinder natives oder chemisch verändertes Hämoglobin oder Myoglobin vom Menschen, Rind, Schwein, Pferd, Ziege, Schaf, oder Mischungen hiervon, in einer wässrigen Salzlösung, vor allem Natriumchloridlösung, vorliegt zum Eintrag in die Bauchhöhle und zur Verteilung/Benetzung zwischen den Bauchhöhlenorganen;
- Zubereitungen mit einer isotonen Lösung mit nativem Schweinehämoglobin;
- Zubereitungen mit nativem oder chemisch verändertem Hämocyanin oder Erythrocruorin eines niederen Tieres in einer wässrigen Salzlösung, vor allem Natriumchloridlösung, zum Eintrag in die Bauchhöhle und zur Verteilung/Benetzung zwischen den Bauchhöhlenorganen;
- Zubereitungen, worin die Konzentration des verwendeten Sauerstoffbinders in der wässrigen Lösung zwischen 150 und 400 g/L, bevorzugt zwischen 200 und 350 g/L und ganz besonders bevorzugt zwischen 250 und 300 g/L beträgt.
- Zubereitungen mit einem oder mehreren Sauerstoffbindern, deren Sauerstoffbindungs-Stellen für eine verbesserte Lagerbarkeit vor der Verwendung mit Kohlenmonoxid gesättigt sind, so dass das Mittel Kohlenmonoxid enthält.
- Zubereitungen, umfassend als Sauerstoffbinder bzw. sauerstoffbindendes Protein natives Hämocyanin oder ein chemisch verändertes Derivat hiervon in einer wässrigen Salzlösung, vor allem Natriumchloridlösung;
- Zubereitungen, umfassend als Sauerstoffbinder bzw. sauerstoffbindendes Protein natives Erythrocruorin oder ein chemisch verändertes Derivat hiervon in einer wässrigen Salzlösung, vor allem Natriumchloridlösung.

2. Verabreichung und Anwendung der Zubereitung

Die erfindungsgemäß eingesetzte Zubereitung wird vorzugsweise vor der Begasung in die Bauchhöhle mittels geeigneter Injektionsvorrichtungen eingebracht.

Die bevorzugte Injektionsgeschwindigkeit der flüssigen Zubereitung hängt ab von der Ausgestaltung des Katheters, der Flüssigkeits-Strahl darf die Bauchorgane nicht verletzen. Zum Beispiel erscheinen etwa 100 - 200 mL/min sinnvoll. Der Eintrag der flüssigen Zubereitung erfolgt aus üblichen Vorratsbehältern, z. B. einer Spritze, einer Infusionsflasche, einem Infusionsbeutel, etc. Vorzugsweise wird erfindungsgemäß der Bauchraum zunächst mit einer bestimmten Menge der flüssigen Zubereitung befüllt bzw. benetzt, und danach eine bestimmte Menge Gas eingefüllt.

Die Begasung der Bauchhöhle ist bekannt, und unterschiedliche Ausrüstungen und Vorgehensweisen sind in der nachfolgend aufgelisteten Literatur beschrieben:

Barr J., et al.: "Peritoneal Ventilation: An Animal Model of Extrapulmonary Ventilation in Experimental Adult Respiratory Distress Syndrome", Pediatric Research 1994; 35: 682 - 284; Zhang J.-Y., et al.: "Effect of Oxygenation of Transperitoneal Ventilation on the Death Time After Asphyxiation in Rabbits", Minerva Anesthesiologica 2010; 76: 913 - 918;
Giffin D.M., et al.: "Oxygen Uptake During Peritoneal Ventilation in a Porcine Model of Hypoxemia", Critical Care Medicine 1998; 26: 1564 - 1568;
Wang X., et al.: "The Effect of High and Conventional Frequency Peritoneal Jet Ventilation on Hypoxemia in ARDS Dogs", European Journal of Anaesthesiology 2012; 29: 180.

Der injizierte Sauerstoffbinder, insbesondere das verabreichte Hämoglobin, vor allem natives Hämoglobin vom Menschen, von Schweinen oder von Rindern, kann durch Ausspülen der Bauchhöhle mit einer Spüllösung mindestens überwiegend wieder entfernt werden. Dies erfolgt über die genannten Katheter. Als Spül-Lösung wird vorzugsweise eine bekannte isotonische wässrige Spüllösung eingesetzt. Somit kann nach Beendigung einer peritonealen Beatmung die Bauchhöhle wieder gereinigt werden. Eine Entfernung des Hämoglobins könnte z. B. auch notwendig werden, um den Sauerstoffbinder, vor allem das Hämoglobin, zu erneuern. Denn eine allmähliche Oxidation des Hämoglobins auch in einer Bauchhöhle zu Methämoglobin (Hämiglobin), das keinen Sauerstoff mehr zu binden vermag, ist wahrscheinlich.

Geeignete Systeme bzw. Vorrichtungen zur Durchführung der Erfindung sind insbesondere aus der Dialysetechnik (Notfall-Peritonealdialyse) hinlänglich bekannt.

Die oben beschriebene wässrige, einen Sauerstoffbinder enthaltende Zubereitung, vor allem mit einem Halbsättigungs-Sauerstoffpartialdruck von 1 bis 50 Torr (mm Hg) ist daher geeignet für die An- und Verwendung zur Verstärkung der Diffusion von in Schleimschichten auf dem Peritoneum eingebrachtem Sauerstoff(gas), und insbesondere zur Benetzung der Bauchhöhle bei einer peritonealen Beatmung für eine verbesserte Sauerstoffdiffusion durch die Schleimschichten auf dem Peritoneum.

Die Anwendung kann wie folgt sein:

i) Bereitstellung einer mindestens einen Sauerstoffbinder, vorzugsweise ausgewählt aus Hämoglobin, Myoglobin, Hämocyanin, Erythrocruorin, in Form nativer oder vernetzter, pegylierter, polymerisierter oder auf andere an sich bekannte Weise chemisch derivatisierter oder karbonylierter Derivate, oder Mischungen hiervon, enthaltenden wässrigen Zubereitung wie oben beschrieben;
ii) Injizieren der Zubereitung in die Bauchhöhle in einer der Anwendung gemäßen Menge und Geschwindigkeit wie oben erläutert;

iii) Begasung der Bauchhöhle in an sich bekannter Weise mit einem Sauerstoffgas-haltigen Gas wie Luft, Stickstoff, enthaltend 25 bis 100 Vol.-% Sauerstoff;

iv) ggf. Abziehen überschüssiger Zubereitung gemäß i) und/oder Spülen der Bauchhöhle mit einer Spüllösung, z. B. isotoner Kochsalzlösung.

3. Indikationen für die erfindungsgemäß eingesetzte Zubereitung

Die Zubereitung ist vor allem geeignet zur Verbesserung der Oxygenierung eines Säugetiers durch eine peritoneale Beatmung bei einer durch ein Lungenversagen induzierten Hypoxie infolge einer bakteriellen oder viralen Infektion, durch Verätzung, bei Kreislauf-Schock wie hämorrhagischer oder septischer Schock, Unfall oder Vergiftung wie z. B. Rauchgasvergiftung, bei einer Lungen-Embolie oder einer Lungenentzündung wegen einer sonstigen Ursache.

Sie kann auch eingesetzt werden zur lokalen Oxygenierung einzelner, an die Bauchhöhle grenzender und zumindest teilweise mit Peritoneum überzogener Organe wie Darm, Magen, Bauchspeicheldrüse und Leber im jeweiligen Bedarfsfall, oder bei einem (relativen oder absoluten) Sauerstoffmangel wegen Minderdurchblutung (lokale Hypoxie), wie z. B. einem lokalen Gefäßverschluss (Infarkt durch Thrombosierung oder Embolie, z. B. Darminfarkt), oder einer Blutfluss-Verteilungsstörung z. B. wegen eines Kreislaufschocks. Ferner kann die erfindungsgemäße Zubereitung auch geeignet sein für eine Steigerung der Oxygenierung von mit Zellen oder Ähnlichem behandelten Organen im Bauchraum wie z. B. bei der Implantation von Insulin-produzierenden Langerhansschen Zellen in die Leber, etc..

Als Patienten kommen vor allem Menschen, Pferde, Kamele, Hunde, Katzen, Rinder oder andere in einer der beschriebenen Bedarfslage befindliche Säugetiere infrage. Insbesondere bezieht sich die erfindungsgemäß eingesetzte Zubereitung auf Menschen.

Ausgestaltungsbeispiele

Beispiel 1 - Gewinnung von und Herstellung einer erfindungsgemäßen Zubereitung mit nativem Schweine-Hämoglobin

Hämoglobin vom Hausschwein wurde aus frischem Blut von Schlachttieren gewonnen und aufgereinigt: Je Liter Schweine-Vollblut wurden 5 mL Phenoxyethanol und 4 g Natriumcitrat addiert und eingemischt. Durch Zentrifugation (12 min · 3.800 g) wurde das Plasma abgetrennt und durch Absaugen entfernt. Die Erythrozyten wurden 2-mal in Kochsalzlösung (9,0 g/L) suspendiert, erneut zentrifugiert und der Überstand abgesaugt. Durch Zugabe von Wasser (2,5 L/L) wurden die Erythrozyten lysiert, durch Zugabe und Einrühren von kristallinem Kochsalz (9,0 g/L) wieder ein isotones Milieu eingestellt, durch Zugabe von NaOH ein pH von

7,0 eingestellt und durch Rühren unter CO-Gas und Durchsprudeln des Gases durch die Hämoglobinlösung das Hämoglobin vollständig karbonyliert. Erhitzen auf 75 °C beseitigte Membranreste der Erythrozyten durch Aggregation, eine Zentrifugation (12 min · 3.800 g) und an-schließende Filtration klärte den Überstand. Danach wurde durch eine dialysierende Ultrafiltration das Medium ausgiebig (2,5 L/L) gegen eine 9,0-g/L-NaCl-Lösung ausgetauscht und mittels reiner Tangentialfluss-Ultrafiltration abschließend die Hämoglobinlösung eingeengt. Zum Auffrischen der Karbonylierung wurde die Lösung erneut mit CO-Gas in Äquilibrium gebracht (durch Schütteln eines je zur Hälfte mit Lösung und CO-Gas gefüllten Behälters).

Es wurde folgende Zubereitung erhalten:

| | |
|---|---|
| Hämoglobin-Konzentration: | 350 g/L |
| NaCl-Konzentration: | 9,0 g/L |
| Kohlenmonoxid-Konzentration: | $\leq$ 1,02 mmol/L (= Löslichkeit im LM) |
| Osmolarität (rechnerisch): | 309 mOsm/L |
| pH: | 7,1 |
| Basis: | Wasser |

Diese Lösung ist geeignet, als erfindungsgemäße Zubereitung verwendet zu werden und die erfindungsgemäße Verbesserung der Sauerstoff-Aufnahme durch eine peritoneale Beatmung zu bewirken.

Beispiel 2 - Herstellung eines vernetzten Schweinehämoglobins

Hochreines Schweinehämoglobin, in einer Konzentration von 330 g/L gelöst in einem wässrigen Elektrolyten der Zusammensetzung 50 mM $NaHCO_3$ und 100 mM NaCl, wurde bei 4 °C durch Rühren der Lösung unter ständig erneuertem, reinen Stickstoff über der Lösung desoxygeniert. Anschließend wurden 4 mol Natrium-Ascorbat (als 1-molare Lösung in Wasser) pro Mol (monomeren) Hämoglobins zugegeben und 6 h reagieren lassen. Die Lösung wurde mit 0,5-molarer Milchsäure auf einen pH-Wert von 7,1 titriert, 1,1 Mol Pyridoxal-5'-Phosphat je Mol Hämoglobin zugegeben und für 16 h reagieren lassen. Nun wurde mit 0,5-molarer Natronlauge ein pH-Wert von 7,8 eingestellt, 1,1 Mol Natriumborhydrid (als 1-molare Lösung in 0,01-molarer Natronlauge) zugegeben und für eine Stunde reagieren lassen. Jetzt wurde mit 0,5-molarer Milchsäure ein pH von 7,3 eingestellt, zunächst 1,1 Mol 2,3-Bisphosphoglyzerat pro Mol Hämoglobin und nach 15 min Reaktionszeit 8 Mol Glutardialdehyd je Mol Hämoglobin, gelöst in 1,8 L reinem Wasser je Liter Hämoglobinlösung zur Vernetzung des Hämoglobins innerhalb 5 Minuten zugegeben und 2,5 h reagieren lassen. Nach Titration mit 0,5-molarer Natronlauge auf einen pH-Wert von 7,8 folgte eine Zugabe von 15 Mol Natriumborhydrid (als 1-molare Lösung in 0,01-molarer Natronlauge) je Mol Hämoglobin für 1 h. Es erfolgte eine Zugabe von 2 Liter Wasser je Liter ursprünglicher Hämoglobinlösung. Der pH-Wert betrug dann 9,3 und es folgte direkt eine Zugabe von 4 Mol Methoxy-Succinimidyl-propionat-Polyethylenglykol des Molekulargewichts 2000 g/mol für 2 h. Die Stickstoffatmosphäre über der Lösung wurde durch reinen Sauerstoff ersetzt.

Nach 1 h wurden unlösliche Bestandteile durch Zentrifugation (20.000 g für 15 min) abgetrennt. Anschließend erfolgte ein Wechsel des Elektrolyten durch eine Volumenausschluss-Chromatographie (Sephadex G-25 - Gel, Pharmacia, D) zu einer wässrigen Elektrolyt-Lösung der Zusammensetzung 125 mM NaCl, 4,5 mM KCl und 20 mM $NaHCO_3$.

Die Ausbeute betrug 77 %, die Ausbeute für Molekulargewicht größer 700 000 g/Mol 28 %. Der Methämoglobin-Anteil betrug unter 5 %, Messungen der Charakteristik der Sauerstoffbindung unter physiologischen Bedingungen (eine Temperatur von 37 °C, ein Kohlendioxid-Partialdruck von 40 Torr und ein pH-Wert von 7,4) ergaben für das Produkt einen p50-Wert von 22 Torr und einen n50-Wert von 1,95.

Dieses vernetzte Schweine-Hämoglobin ist geeignet, in einer erfindungsgemäßen Zubereitung zur Verbesserung der Sauerstoff-Aufnahme durch eine peritoneale Beatmung verwendet zu werden.

Analog den oben genannten Beispielen können auch erfindungsgemäße Zubereitungen mit nativem oder vernetztem Myoglobin gemäß Beispiele 1 bzw. 2 oder mit Hämocyanin (nativ) in der angegebenen bevorzugten Menge von 150 bis 400 g/L hergestellt und angewendet werden.

Beispiel 3 - Verbesserung einer peritonealen Beatmung

Eine erfindungsgemäße verbesserte Gewebe-Oxygenierung durch eine peritoneale Beatmung wird durch eine verbesserte Oxygenierung des hypoxischen Ileums von beatmeten, narkotisierten Ratten mit einem experimentell erzeugten, septischen Schock demonstriert, wobei eine Zubereitung gemäß dem Beispiel 1 verwendet wurde.

In Ratten (mit Körpergewichten um 300 g) wurde zunächst ein septischer Schock durch eine i.v.-Injektion von LPS (Lipopolysaccharide, etwa 3 mg/kg) von Escherichhia coli erzeugt. Nach 3 Stunden wurden die Tiere mit Isofluran narkotisiert. Um einen stabilen systemischen Blutdruck um etwa 90 mmHg generieren und aufrecht zu erhalten, erhielten die Tiere Noradrenalin infundiert (je nach Bedarf zwischen 0,01 und 10 mg $kg^{-1}$ $min^{-1}$ i.v.).

Ein Reflexionsspektrometer O2C (LEA Medizintechnik, Giessen, Deutschland) diente zur Ermittlung der Oxygenierung (in Form der Sauerstoffsättigung des Hämoglobins in den Ratten-Erythrocyten in den Kapillaren und Venulen) des Ileums, diese diente als Maß für die Effektivität der peritonealen Beatmung. Die Messsonde wurde auf das Ileum gelegt und fixiert. Um die erfindungsgemäße Verbesserung der Oxygenierung des Ileums zu demonstrieren, wurde die abdominale Serosa der Tiere entweder mit 0,5 mL wässriger, isotoner NaCl-Lösung (Vergleichs-Gruppe) oder mit 0,5 mL einer wässrigen Lösung von 20 % Hämoglobin in isotoner NaCl-Lösung (Interventions-Gruppe) benetzt und nachfolgend reines (befeuchtetes) Sauerstoffgas (99,5 %) langsam (etwa 5 mL/min) durch die Leibeshöhle strömen gelassen. Tiere, deren Serosa mit der Hämoglobin-Lösung benetzt war, zeigten, verglichen mit den Tieren der Vergleichs-Gruppe, eine höhere Sauerstoffsättigung im Blut des Ileums.

**Patentansprüche**

1. Flüssige, ein Sauerstoff bindendes Protein, ausgewählt Hämoglobin, Myoglobin, Hämocyanin, Erythrocruorin oder Mischungen hiervon, enthaltende wässrige Zubereitung zur Verwendung als ein die Diffusion des bei der peritonealen Beatmung eingesetzten Sauerstoff enthaltenden Gases verstärkendes Benetzungsmittel der Bauchhöhle zur Verbesserung der Oxygenierung durch peritoneale Beatmung eines Säugetiers im hypoxischen Bedarfsfall.

2. Zubereitung zur Verwendung nach Anspruch 1, **dadurch gekennzeichnet, dass** Hämoglobin, Myoglobin, Hämocyanin, Erythrocruorin oder Mischungen hiervon, ausgewählt sind aus nativem oder chemisch verändertem Hämoglobin, Myoglobin, Hämocyanin, Erythrocruorin oder Mischungen hiervon, wobei die chemische Veränderung eine Vernetzung, eine Polymerisation, eine kovalente Addition eines weiteren Moleküls oder eine Kombination einer oder mehrerer dieser Modifikationen sein kann.

3. Zubereitung zur Verwendung nach Anspruch 1 oder 2, **dadurch gekennzeichnet, dass** als Sauerstoffbindendes Protein (Sauerstoffbinder) natives oder chemisch verändertes Hämoglobin oder Myoglobin vom Menschen, Rind, Schwein, Pferd, Ziege, Schaf, oder Mischungen hiervon, in einer wässrigen Salzlösung zum Eintrag in die Bauchhöhle und zur Verteilung zwischen den Bauchhöhlenorganen sowie zur Benetzung von deren Oberfläche eingesetzt wird.

4.  Zubereitung zur Verwendung nach einem der Ansprüche 1 bis 3, **dadurch gekennzeichnet, dass** eine isotone Lösung mit nativem Schweinehämoglobin eingesetzt wird.

5.  Zubereitung zur Verwendung nach Anspruch 1 oder 2, **dadurch gekennzeichnet, dass** als Sauerstoffbindendes Protein (Sauerstoffbinder) ein natives oder chemisch verändertes Hämocyanin oder Erythrocruorin eines niederen Tieres in einer wässrigen Salzlösung zum Eintrag in die Bauchhöhle und zur Verteilung zwischen den Bauchhöhlenorganen sowie zur Benetzung von deren Oberfläche eingesetzt wird.

6.  Zubereitung zur Verwendung nach einem der Ansprüche 1 bis 5, **dadurch gekennzeichnet, dass** die Konzentration des verwendeten Sauerstoffbindenden Proteins (Sauerstoffbinders) in der wässrigen Lösung 150 bis 400 g/l beträgt.

7.  Zubereitung zur Verwendung nach einem der Ansprüche 1 bis 5, **dadurch gekennzeichnet, dass** für eine verbesserte Lagerbarkeit vor der Verwendung die Sauerstoffbindungs-Stellen des Sauerstoffbindenden Proteins (Sauerstoffbinders) mit Kohlenmonoxid gesättigt sind, so dass das Mittel Kohlenmonoxid enthält.

8.  Zubereitung zur Verwendung nach einem der Ansprüche 1 bis 7 in Kombination mit einem in die Peritonealhöhle verabreichten Sauerstoff-haltigen Gas zur verbesserten peritonealen Beatmung bei akuter Hypoxie infolge Lungenversagens.

9.  Zubereitung zur Verwendung nach einem der Ansprüche 1 bis 7 oder nach Anspruch 8 zur systemischen Verbesserung der Oxygenierung eines Säugetiers durch peritoneale Beatmung bei einer durch Lungenversagen induzierten Hypoxie in einem Säuger infolge einer bakteriellen oder viralen Infektion, einer sonstigen Lungenentzündung, eines insbesondere hämorrhagischen oder septischen Kreislauf-Schocks, durch Verätzung, Unfall, Vergiftung (z. B. Rauchvergiftung) oder einer Lungen-Embolie.

10. Zubereitung zur Verwendung nach einem der Ansprüche 1 bis 7 oder nach Anspruch 8 zur lokalen Verbesserung der Oxygenierung einzelner, an die Bauchhöhle angrenzender und zumindest teilweise mit Peritoneum überzogener Organe wie Darm, Magen, Leber, Bauchspeicheldrüse (etc.) im jeweiligen Bedarfsfall wie einem (relativen oder absoluten) Sauerstoffmangel wegen Minderdurchblutung, wie z. B. einem lokalen Gefäßverschluss (Infarkt durch Thrombosierung oder Embolie, z. B. Darminfarkt), oder einer Blutfluss-Verteilungsstörung z. B. wegen eines Kreislaufschocks, etc.

11. Zubereitung zur Verwendung nach einem der Ansprüche 1 bis 10, **dadurch gekennzeichnet, dass** als Vernetzung eine Vernetzung mit Divinylsulfon, Epichlorhydrin, Butadiendiepoxid, Hexamethylendiisocyanat, Dialdehyden wie Glyoxal und Glutardialdehyd, Diimidoestern wie Dimethylsuberimidat, Dimethylmalonimidat und Dimethyladipimidat, vorliegt.

12. Zubereitung zur Verwendung nach einem der Ansprüche 1 bis 11, **dadurch gekennzeichnet, dass** als kovalente Anknüpfung eine Pegylierung mit Polyethylenoxid, Polypropylenoxid, oder Kopolymeren aus Ethylenoxid und Propylenoxid, vorliegt.

## Claims

1.  Liquid aqueous composition containing an oxygen-binding protein selected from haemoglobin, myoglobin, haemocyanin, erythrocruorin or mixtures thereof, for use as an abdominal cavity wetting agent enhancing diffusion of the oxygen-containing gas used in peritoneal ventilation in order to improve oxygenation by peritoneal ventilation of a mammal in case of hypoxic need.

2.  Composition for use according to claim 1, **characterized in that** haemoglobin, myoglobin, haemocyanin, erythrocruorin or mixtures thereof are selected from native or chemically modified haemoglobin, myoglobin, haemocyanin, erythrocruorin or mixtures thereof, where said chemical modification may be a cross-linking, a polymerisation, a covalent addition of another molecule or a combination of any of these modifications.

3. Composition for use according to claim 1 or 2, **characterized in that** native or chemically modified haemoglobin or myoglobin of human, bovine, porcine, equine, caprine or ovine origin, or mixtures thereof, in an aqueous salt solution for introduction into the abdominal cavity and for distribution between the organs of the abdominal cavity and the wetting of their surface is used as the oxygen-binding protein (oxygen carrier).

4. Composition for use according to any of claims 1 to 3, **characterized in that** an isotonic solution comprising native porcine haemoglobin is used.

5. Composition for use according to claim 1 or 2, **characterized in that** native or chemically modified haemocyanin or erythrocruorin of a lower animal in an aqueous salt solution for introduction into the abdominal cavity and for distribution between the organs of the abdominal cavity and the wetting of their surface is used as the oxygen-binding protein (oxygen carrier).

6. Composition for use according to any of claims 1 to 5, **characterized in that** the concentration of the oxygen-binding protein (oxygen carrier) used in the aqueous solution amounts to 150 to 400 g/l.

7. Composition for use according to any of claims 1 to 5, **characterized in that** the oxygen-binding sites of the oxygen-binding protein (oxygen carrier) are saturated with carbon monoxide for improved shelf life prior to use, so the agent comprises carbon monoxide.

8. Composition for use according to any of claims 1 to 7 in combination with an oxygen oxygen-comprising gas administered into the peritoneal cavity for improved peritoneal ventilation in acute hypoxia due to pulmonary failure.

9. Composition for use according to any of claims 1 to 7 or according to claim 8 for systemic improvement of the oxygenation of a mammal by peritoneal ventilation in case of hypoxia induced by pulmonary failure in a mammal due to bacterial or viral infection, to any other pneumonia, to circulatory shock of a preferably haemorrhagic or septic nature, to corrosive injury, to trauma, to intoxication (e.g. smoke inhalation), or to pulmonary embolism.

10. Composition for use according to any of claims 1 to 7 or according to claim 8 for local improvement of the oxygenation of individual organs adjacent to the abdominal cavity and at least partially covered with peritoneum such as intestines, stomach, liver, pancreas (etc.) in case of relevant need such as (relative or absolute) oxygen deficiency due to reduced perfusion, e.g. local vascular occlusion (infarction due to thrombosis or embolism, e.g. bowel infarction), or a blood flow distribution disorder, e.g. resulting from circulatory shock, etc.

11. Composition for use according to any of claims 1 to 10, **characterized in that** a cross-linking with divinyl sulphone, epichlorohydrin, butadiene diepoxide, hexamethylene diisocyanate, dialdehydes such as glyoxal and glutardialdehyde, diimido esters such as dimethyl suberimidate, dimethyl malonimidate and dimethyl adipimidate is present as cross-linking.

12. Composition for use according to any of claims 1 to 11, **characterized in that** pegylation with polyethylene oxide, polypropylene oxide or copolymers of ethylene oxide and propylene oxide is present as covalent linkage.

**Revendications**

1. Préparation aqueuse liquide contenant une protéine liant l'oxygène, sélectionnée à partir d'hémoglobine, de myoglobine, d'hémocyanine, d'érythrocruorine ou de mélanges de celles-ci, pour utilisation comme agent mouillant de la cavité abdominale renforçant la diffusion du gaz contenant l'oxygène utilisé lors de la ventilation péritonéale afin d'améliorer l'oxygénation par ventilation péritonéale d'un mammifère présentant une urgence hypoxique.

2. Préparation pour utilisation selon la revendication 1, **caractérisée par le fait que** l'hémoglobine, la myoglobine, l'hémocyanine, l'érythrocruorine ou les mélanges de celles-ci, sont sélectionnées à partir d'hémoglobine, de myoglobine, d'hémocyanine, d'érythrocruorine natives ou modifiées chimiquement ou de mélanges de celles-ci, la modification chimique pouvant être une réticulation, une polymérisation, une addition covalente d'une autre molécule ou une combinaison de plusieurs de ces modifications.

3. Préparation pour utilisation selon la revendication 1 ou 2, **caractérisée par le fait qu'**en tant que protéine liant l'oxygène (liant d'oxygène), de l'hémoglobine ou de la myoglobine native ou modifiée chimiquement humaine, bovine, porcine, équine, caprine, ovine ou de mélanges de celles-ci, est utilisée dans une solution saline aqueuse pour l'application dans la cavité abdominale et pour la répartition entre les organes de la cavité abdominale ainsi que pour le mouillage de leur surface.

4. Préparation pour utilisation selon l'une des revendications 1 à 3, **caractérisée par le fait qu'**une solution isotonique avec de l'hémoglobine native porcine est utilisée.

5. Préparation pour utilisation selon la revendication 1 ou 2, **caractérisée par le fait qu'**en tant que protéine liant l'oxygène (liant d'oxygène), de l'hémocyanine ou de l'érythrocruorine native ou modifiée chimiquement d'un animal inférieur est utilisée dans une solution saline aqueuse pour l'application dans la cavité abdominale et pour la répartition entre les organes de la cavité abdominale ainsi que pour le mouillage de leur surface.

6. Préparation pour utilisation selon l'une des revendications 1 à 5, **caractérisée par le fait que** la concentration de la protéine utilisée liant l'oxygène (liant d'oxygène) est de 150 à 400 g/l dans la solution aqueuse.

7. Préparation pour utilisation selon l'une des revendications 1 à 5, **caractérisée par le fait que** pour une meilleure stockabilité avant l'utilisation, les sites de liaison de l'oxygène de la protéine liant l'oxygène (liant d'oxygène) doivent être saturés avec du monoxyde de carbone, de manière à ce que le produit contienne du monoxyde de carbone.

8. Préparation pour utilisation selon l'une des revendications 1 à 7 en association à un gaz contenant de l'oxygène administré dans la cavité péritonéale pour une meilleure ventilation péritonéale en cas d'hypoxie aiguë suite à une défaillance pulmonaire.

9. Préparation pour utilisation selon l'une des revendications 1 à 7 ou selon la revendication 8 pour l'amélioration systémique de l'oxygénation d'un mammifère par ventilation péritonéale en cas d'hypoxie induite par une défaillance pulmonaire chez un mammifère suite à une infection bactérienne ou virale, toute autre forme de pneumonie, un choc cardio-vasculaire - en particulier hémorragique ou septique - dû à une corrosion/brûlure, un accident, une intoxication (p. ex. intoxication par la fumée) ou une embolie pulmonaire.

10. Préparation pour utilisation selon l'une des revendications 1 à 7 ou selon la revendication 8 pour l'amélioration locale de l'oxygénation de différents organes voisins de la cavité abdominale et d'organes au moins recouverts partiellement de péritoine tels que l'intestin, l'estomac, le foie, le pancréas (etc.) dans l'urgence en question comme un manque d'oxygène (relatif ou absolu) à cause d'une diminution de l'irrigation sanguine, comme p. ex. une occlusion vasculaire locale (infarctus dû à une thrombose ou une embolie, p. ex. infarctus intestinal) ou un trouble de la diffusion du flux sanguin, p. ex. à cause d'un choc cardio-vasculaire, etc.

11. Préparation pour utilisation selon l'une des revendications 1 à 10, **caractérisée par** la présence d'une réticulation avec de la divinyl sulfone, de l'épichlorhydrine, du butadiène diépoxyde, du diisocyanate d'hexaméthylène, des dialdéhydes tels que le glyoxal et le glutaraldéhyde, des diimidoesters tels que le diméthylsubérimidate, le diméthylmalonimidate et le diméthyladipimidate.

12. Préparation pour utilisation selon l'une des revendications 1 à 11, **caractérisée par** la présence d'une liaison covalente sous forme de pégylation avec l'oxyde de polyéthylène, l'oxyde de polypropylène, ou des copolymères à base d'oxyde de polyéthylène et d'oxyde de propylène.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4657532 A
- US 4963130 A
- EP 0209128 B1
- DE 10031740 A1
- DE 10031744 A1
- DE 10031742 A1

**In der Beschreibung aufgeführte Nicht-Patentliteratur**

- **ZHANG J.-Y. et al.** Effect of Oxygenation of Transperitoneal Ventilation on the Death Time After Asphyxiation in Rabbits. *Minerva Anesthesiologica,* 2010, vol. 76, 913-918
- **GIFFIN D.M. et al.** Oxygen Uptake During Peritoneal Ventilation in a Porcine Model of Hypoxemia. *Critical Care Medicine,* 1998, vol. 26, 1564-1568
- **J. B. WITTENBERG.** The Molecular Mechanism of Haemoglobin-facilitated Oxygen Diffusion. *Journal of Biological Chemistry,* 1966, vol. 241, 104-114
- **BARR J. et al.** Peritoneal Ventilation: An Animal Model of Extrapulmonary Ventilation in Experimental Adult Respiratory Distress Syndrome. *Pediatric Research,* 1994, vol. 35, 682-284
- **WANG X. et al.** The Effect of High and Conventional Frequency Peritoneal Jet Ventilation on Hypoxemia in ARDS Dogs. *European Journal of Anaesthesiology,* 2012, vol. 29, 180